(19) Europäisches Patentamt
European Patent Office
Office européen des brevets

(11) EP 0 610 138 B1

(12) **FASCICULE DE BREVET EUROPEEN**

(45) Date de publication et mention de la délivrance du brevet:
**31.07.1996 Bulletin 1996/31**

(51) Int. Cl.[6]: **C07J 71/00**, C07J 3/00

(21) Numéro de dépôt: **94400227.8**

(22) Date de dépôt: **03.02.1994**

(54) **Nouveau procédé de préparation de stéroides 6alpha,9alpha-difluorés et nouveaux intermédiaires obtenus**

Neues Verfahren zur Herstellung von 6alpha, 9alphadifluoro-Sterioden und Zwischenprodukte davon

New process for preparation of 6alpha,9alpha difluoro-steroids and intermediates therefor

(84) Etats contractants désignés:
**AT BE CH DE DK ES FR GB GR IE IT LI LU NL PT SE**

(30) Priorité: **05.02.1993 FR 9301275**

(43) Date de publication de la demande:
**10.08.1994 Bulletin 1994/32**

(73) Titulaire: **ROUSSEL UCLAF**
**F-93230 Romainville (FR)**

(72) Inventeurs:
- **Godard, Jean-Yves**
  **F-93340 Le Raincy (FR)**
- **Mackiewicz, Philippe**
  **F-93190 Livry Gargan (FR)**
- **Prat, Denis**
  **F-93500 Pantin (FR)**
- **Richard, Christian**
  **F-93110 Rosny sous Bois (FR)**

(74) Mandataire: **Vieillefosse, Jean-Claude et al**
**ROUSSEL UCLAF,**
**Département des Brevets,**
**102, Route de Noisy**
**93235 Romainville Cédex (FR)**

(56) Documents cités:
**EP-A- 0 100 874**
**EP-A- 0 135 476**
**WO-A-91/19731**
**FR-A- 2 026 919**
**FR-A- 2 424 286**
**GB-A- 2 018 258**
**US-A- 4 198 336**

EP 0 610 138 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet un nouveau procédé de préparation de stéroïdes 6α,9α-difluorés et les nouveaux intermédiaires obtenus.

L'invention a ainsi pour objet un procédé de préparation des composés de formule (I) :

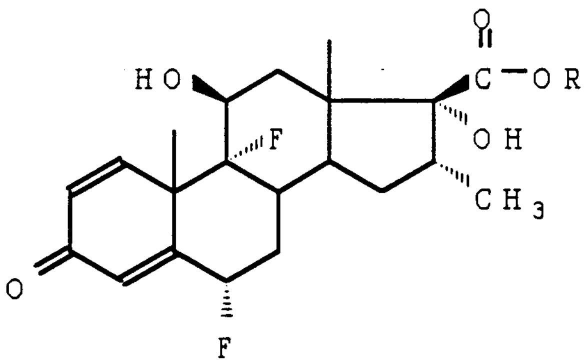

(I)

dans laquelle R représente un atome d'hydrogène ou un reste ester, caractérisé en ce que l'on traite un composé de formule (II) :

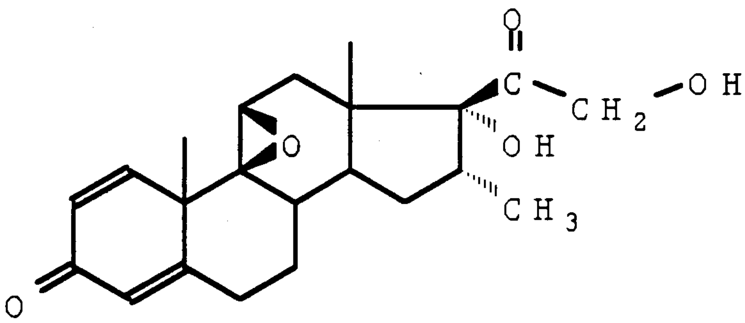

(II)

par un agent de dégradation oxydant, pour obtenir un composé de formule (III) :

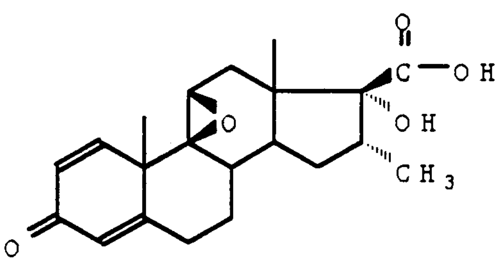

(III)

dont on protège la fonction cétone en 3 sous forme d'éther ou d'ester d'énol et, le cas échéant, la fonction acide en 17β sous forme d'ester, pour obtenir un composé de formule (IV) :

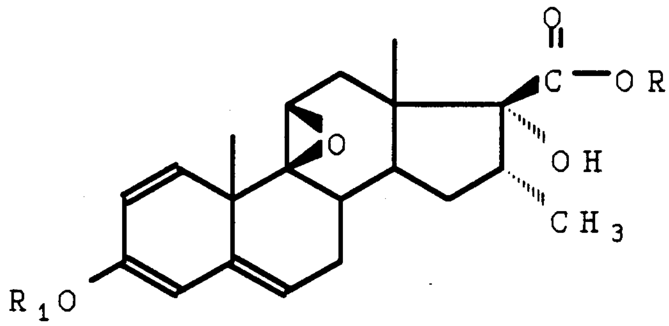

(IV)

dans laquelle R est défini comme précédemment et $R_1$ représente un reste d'éther ou d'ester d'énol, sur lequel on fait agir un agent de fluoration électrophile, pour obtenir un composé de formule (V) :

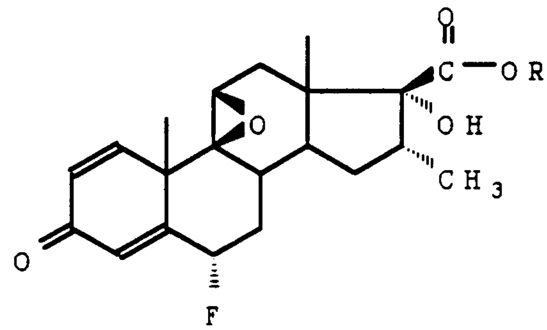

(V)

que l'on traite par un agent de fluoration nucléophile, pour obtenir le composé de formule (I) attendu, que, le cas échéant, lorsque R représente un reste ester, l'on saponifie pour obtenir l'acide correspondant.

Par reste ester on entend tout reste connu de l'homme du métier et notamment un radical alkyle renfermant de 1 à 6 atomes de carbone, un radical aryle renfermant de 6 à 10 atomes de carbone ou un radical aralkyle renfermant de 7 à 12 atomes de carbone.

Lorsque R représente un radical alkyle, il s'agit par exemple d'un radical méthyle, éthyle, propyle, isopropyle, n-butyle, sec-butyle, tert-butyle, pentyle ou hexyle.

Lorsque R représente un radical aryle, il s'agit par exemple d'un radical phényle ou d'un radical phényl substitué, en particulier par un ou plusieurs radicaux alkyles.

Lorsque R représente un radical aralkyle, il s'agit par exemple d'un radical benzyle ou phénéthyle.

Par reste ester, on entend également un reste silylé, par exemple d'un reste trialkylsilyle, tel que triméthylsilyle, tert-butyl diméthylsilyle ou encore par exemple d'un reste triarylsilyle tel que triphénylsilyle ou diarylalkylsilyle tel que diphényl tert-butylsilyle.

Par reste d'éther d'énol en position 3, on entend tout reste connu de l'homme du métier pour bloquer cette position 3 sous cette forme et notamment un radical alkyle renfermant de 1 à 6 atomes de carbone, par exemple méthyle, éthyle ou propyle, un radical benzyle, un radical tétrahydropyranyle, ou un groupement silylé, par exemple l'un de ceux mentionnés plus haut.

Par reste d'ester d'énol en position 3, on entend un reste de formule -COR, R étant un radical alkyle tel que défini ci-dessus, ou un radical aryle ou aralkyle, tel que défini ci-dessus, et pouvant être substitué par un ou plusieurs radicaux nitro, halogéno, notamment chloro, ou alkyles renfermant de 1 à 4 atomes de carbone.

L'invention a notamment pour objet un procédé tel que défini précédemment, caractérisé en ce que l'on protège d'abord la fonction acide du composé de formule (III), pour obtenir un composé de formule (III') :

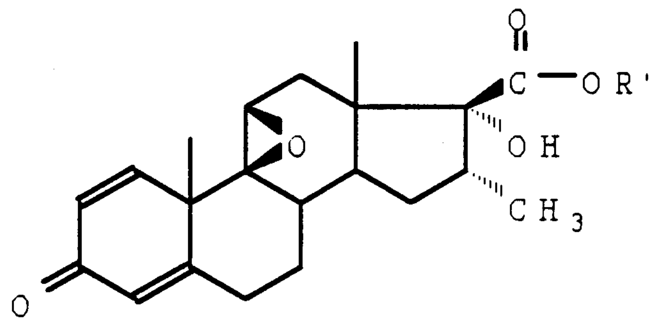

(III')

dans laquelle R' représente un reste ester, dont on protège ensuite la fonction cétone en 3 sous forme d'éther ou d'ester d'énol, pour obtenir un composé de formule (IV) telle que définie précédemment, dans laquelle R a la définition de R' indiquée ci-dessus et poursuit la synthèse comme indiqué précédemment.

La protection de la fonction acide en 17 peut être effectuée par l'un ou l'autre des restes esters mentionnés plus haut, un reste alkyle et notamment méthyle et éthyle étant plus particulièrement préféré.

La protection de la fonction cétone en 3 peut être effectuée par l'un ou l'autre des restes éthers ou esters mentionnés plus haut, un reste ester étant plus particulièrement préféré. On peut citer notamment un reste benzoyle éventuellement substitué par un ou plusieurs radicaux nitro, chloro ou méthyle, ou un reste acétyle, propionyle, butyryle ou encore valéryle.

L'invention a aussi notamment pour objet un procédé tel que défini précédemment, caractérisé en ce que l'on protège dans une même opération la fonction cétone en 3 et la fonction acide en 17β respectivement sous forme d'éther d'énol et d'ester, pour obtenir un composé de formule (IV) telle que définie précédemment, dans laquelle R et $R_1$ représentent un même groupement protecteur et poursuit la synthèse comme indiqué précédemment.

La protection en 3 et 17 est alors de préférence réalisée respectivement sous forme d'éther et d'ester silylés, tels que définis plus haut.

L'invention a encore notamment pour objet un procédé tel que défini précédemment, caractérisé en ce que l'on protège seulement la fonction cétone en 3 sous forme d'éther ou d'ester d'énol, pour obtenir un composé de formule (IV) telle que définie précédemment, dans laquelle R représente un atome d'hydrogène, puis poursuit la synthèse comme indiqué précédemment.

La protection en 3 est alors de préférence réalisée sous forme d'ester d'énol, notamment l'un de ceux mentionnés plus haut.

L'agent de dégradation oxydant utilisé dans le procédé selon l'invention peut être par exemple l'acide periodique, le tétracétate de plomb, le permanganate de potassium, l'eau oxygénée, l'acide periodique catalytique utilisé en présence d'eau oxygénée, les persulfates alcalins tels que l'oxone® (sel triple $2KHSO_5$-$KHSO_4$-$K_2SO_4$) ou le monopersulfate de potassium. L'acide periodique est plus particulièrement préféré.

Les divers blocages en 3 et 17 évoqués plus haut, c'est-à-dire le choix des réactifs correspondants et leurs mises en oeuvre sont à la portée de l'homme du métier. Le blocage sous forme d'ester en 17 pourra par exemple être effectué par action d'un alcool en milieu acide ou en présence de dicyclohexylcarbodiimide et de diméthylaminopyridine, ou encore, dans le cas d'un ester méthylique, par action du diazométhane, du sulfate de méthyle ou du carbonate de méthyle.

Il peut être avantageux d'opérer en phases hétérogènes en présence d'un catalyseur de transfert de phase, lequel peut être notamment un sel d'ammonium quaternaire, par exemple le bromure de tétrabutylammonium, le chlorure de triéthylbenzylammonium ou le chlorure de tricaprylméthylammonium, ou un sel de phosphonium. Le solvant organique utilisé peut être un solvant chloré, par exemple le chloroforme, le chlorure de méthylène ou encore le dichlorométhane, un solvant aromatique, par exemple le toluène, le xylène ou le benzène, ou un solvant aliphatique ou cycloaliphatique, par exemple l'hexane ou le cyclohexane.

Le blocage sous forme d'ester d'énol en 3 pourra par exemple être effectué par action d'un chlorure d'acide approprié, en opérant en présence d'une base azotée ou par trans-estérification à l'aide d'un ester d'énol, par exemple un acétate de type

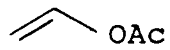 ou 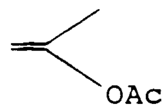

Le blocage sous forme d'éther d'énol pourra quant à lui être effectué par action d'un halogénure d'alkyle en milieu alcalin, ou encore par action du dihydropyranne, d'un ortho-formiate d'alkyle ou d'un alcool en milieu acide.

Les blocages en 17 sous forme d'ester et en 3 sous forme d'éther d'énol peuvent encore être réalisés dans une même opération, tout particulièrement dans le cas d'un blocage sous forme d'ester et d'éther silylés, par action d'un halogénure approprié. Il est à noter que dans un tel cas, le caractère particulièrement labile de l'ester silylé entrainera le retour à l'acide dès l'hydrolyse qui suit la fluoration en 6.

L'agent de fluoration utilisé dans le procédé selon l'invention doit être, comme indiqué plus haut, un agent électrophile.

On peut citer en particulier le fluorure de perchloryle, le fluorure de trifluorométhane sulfonyle et ses dérivés, le N-fluoropyridinium pyridine heptafluorodiborate, l'hypofluorite d'acétyle ou de trifluoroacétyle, le N-fluoropyridinium, les N-fluoro sulfonamides ou N-fluoro sulfonimides, par exemple le N-fluorobenzène sulfonimide ou, de préférence, le Sélectfluor® ou N-fluoro, N-chlorométhyl triéthylène diamine bis tétrafluoroborate. On opère en milieu solvant, par exemple au sein du tétrahydrofuranne, de l'acétone, du chlorure de méthylène, du toluène, et, en particulier dans le cas de la fluoration par le Sélectfluor®, au sein d'un solvant polaire protique ou non, par exemple le diméthylformamide, le méthanol ou, de préférence, l'acétonitrile et, de préférence également, en présence d'eau. La température de la réaction est soit la température ambiante, soit une température inférieure. On peut opérer en présence d'un catalyseur de transfert de phase, notamment l'un de ceux mentionnés plus haut, et, le cas échéant, d'un cosolvant, notamment un solvant chloré tel que ceux mentionnés plus haut.

La fluoration électrophile en position 6 entraîne après hydrolyse le déblocage de la cétone en 3 et le retour au système de doubles liaisons Δ1,4.

L'agent de fluoration nucléophile que l'on fait agir sur le composé de formule (V) peut notamment être l'acide fluorhydrique, en particulier l'acide fluorhydrique aqueux, ou le complexe de l'acide fluorhydrique avec le tétrahydrofuranne, ou, de préférence, le diméthylformamide. On opère par exemple à une température comprise entre -10 et +25°C, avec ou de préférence sans la présence d'un cosolvant.

La saponification finale éventuelle est effectuée selon des techniques connues de l'homme du métier. On peut citer l'hydrolyse ou une alcoolyse en présence d'une base, par exemple un hydroxyde de métal alcalin ou alcalinoterreux ou une base azotée appropriée.

L'invention a aussi pour objet, à titre de composés industriels nouveaux et notamment à titre d'intermédiaires nécessaires à la mise en oeuvre du procédé ci-dessus, les composés de formule (VI) :

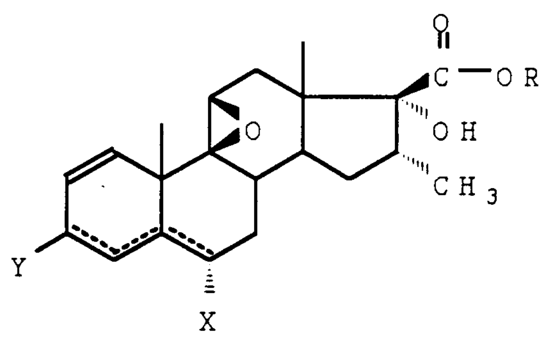

(VI)

dans laquelle R est défini comme précédemment, X représente un atome d'hydrogène ou de fluor et Y associé aux traits pointillés représente un système 3-céto Δ4 ou X représente un atome d'hydrogène et Y associé aux traits pointillés représente un système 3-$OR_1$ Δ3,5, $R_1$ étant défini comme précédemment.

Le composé de formule (II) est décrit dans le brevet US 3 947 409.

Les composés de formule (I)sont décrits par exemple, dans le brevet français 2 026 919 aussi que leur production par la degradation oxidative das produits 6α,9α-difluoro pregnanes correspondants. Le document FR-A-2,424,286 décrit la production des dérivés 6α,9α-difluoro pregnanes par fluorination électrophile et nucléophile des composés 9,11-β-époxy pregnanes correspondants. Les esters possèdent en particulier des propriétés anti-inflammatoires.

Les exemples suivantes illustrent l'invention sans toutefois la limiter.

**EXAMPLE 1 : 6α,9α-difluoro 11β,17α-dihydroxy 16α-méthyl 17β-méthoxycarbonyl androsta 1,4-dièn-3-one**

STADE A : 9,11β-époxy 16α-méthyl 17α-hydroxy 17β-carboxy androsta 1,4-dièn-3-one

On mélange 200 g de 9,11β-époxy 16α-méthyl 17α,21-dihydroxy 20-céto pregna 1,4-dièn-3-one et 800 $cm^3$ de méthanol, puis ajoute à 40°C maximum sous agitation en 20 minutes, 128,5 g d'acide orthoperiodique. On maintient la suspension sous agitation sous gaz inerte à 23°/25°C, pendant 1 heure puis la verse dans un mélange de 1000 $cm^3$ d'eau, 2000 g de glace et 200 g de métabisulfite de sodium en 5 minutes. On maintient ensuite sous agitation à 0°/+10°C pendant 30 minutes, filtre et rince à l'eau. On obtient après séchage 192 g de produit attendu utilisé tel quel pour le stade suivant.
Spectre IR ($CHCl_3$),
Absorptions à 3600 $cm^{-1}$ : OH ; 1706 $cm^{-1}$ : C=O acide ; 1662, 1623, 1607 $cm^{-1}$ : céto-3 Δ1,4.
Spectre RMN ($CDCl_3$, 300 MHz, ppm)
0,97 (d) : 16-$CH_3$ ; 1,09 (s) : 18-$CH_3$ : 1,45 (s) : 19-$CH_3$ ; 3,21 (s) : $H_{11}$ ; 3,94 : OH ; 6,19 (s) : $H_4$ ; 6,23 (dd) : $H_2$ ; 6,64 (d) : $H_1$.

STADE B : 9,11β-époxy 16α-méthyl 17α-hydroxy 17β-méthoxycarbonyl androsta 1,4-dièn-3-one

On mélange 192 g de produit obtenu au stade A, 800 $cm^3$ de chlorure de méthylène et 4 g de bromure de tétrabutylammonium. On introduit sous gaz inerte en 5 minutes environ à +18°/+22°C, 400 $cm^3$ de soude 2N, puis 45,8 $cm^3$ de sulfate diméthylique. On maintient sous agitation pendant 1 heure 30 minutes, puis décante et réextrait la phase aqueuse au chlorure de méthylène. On lave les phases organiques réunies à l'eau, concentre à environ 400 $cm^3$, puis poursuit la distillation en remplaçant le chlorure de méthylène par de l'éther isopropylique. On laisse en 1 heure refroidir à température ambiante en maintenant l'agitation, puis maintient à nouveau 1 heure dans ces conditions et essore puis rince les cristaux à l'éther isopropylique et les sèche. On obtient 185,4 g de produit attendu.
Spectre IR ($CHCl_3$),
Absorptions à 3600 et 3540 $cm^{-1}$ : OH ; 1743, 1713 et 1438 $cm^{-1}$ : $CO_2Me$ ; 1662, 1624 et 1608 $cm^{-1}$ : céto-3 Δ1,4.
Spectre RMN ($CDCl_3$, 300 MHz, ppm)
0,93 (d) : 16-$CH_3$ ; 0,98 (s) : 18-$CH_3$ ; 1,44 (s) : 19-$CH_3$ : 2,97 (s) : OH ; 3,21 (t) : $H_{11}$ ; 3,77 (s) : $CO_2CH_3$ ; 6,15 (s): $H_4$ ; 6,19 (dd) : $H_2$ ; 6,61 (d) : $H_1$.

| Analyse ($C_{22}H_{28}O_5$ : 372,5) | | |
|---|---|---|
| | C % | H % |
| Calculé | 70,9 | 7,6 |
| Trouvé | 71,0 | 7,8 |

STADE C : 3-benzoyloxy 9,11β-époxy 16α-méthyl 17α-hydroxy 17β-méthoxycarbonyl androsta 1,3,5-triène

On mélange à +20°/+22°C, sous gaz inerte, 30 g de produit obtenu au stade B, 75 mg d'hydroquinone et 42 $cm^3$ de pyridine, chauffe à 70°C et ajoute 13 $cm^3$ de chlorure de benzoyle. On maintient à 70°C pendant 6 heures puis laisse revenir à 40°C. On ajoute 30 $cm^3$ de méthanol, maintient sous agitation à 40°C pendant 30 minutes puis laisse revenir à température ambiante. On verse la solution dans un mélange de 300 $cm^3$ d'eau et 44 $cm^3$ d'acide chlorhydrique à 22°Bé, rajoute 270 $cm^3$ de méthanol et maintient sous agitation pendant 1 heure. On essore les cristaux, les lave à l'eau et les sèche. On obtient 36,95 g de produit attendu que l'on peut purifier en le dissolvant dans 2 volumes de chlorure de méthylène, en y ajoutant 5 volumes de méthanol et en distillant le chlorure de méthylène. Après retour à température ambiante sous agitation puis 1 heure à 0°C, on isole 24,97 g de produit attendu sec.
Spectre IR ($CHCl_3$),
Absorptions à 3600 et 3540 $cm^{-1}$ : OH ; 1730 $cm^{-1}$ : C=O ; 1438 $cm^{-1}$ : $OCH_3$ ; 1657, 1620, 1603 et 1585 $cm^{-1}$ : C=C aromatiques.
Spectre RMN ($CDCl_3$, 300 MHz, ppm)
0,94 (d, J = 7) : 16-$CH_3$ ; 0,98 (s) : 18-$CH_3$ ; 1,28 (s) : 19-$CH_3$ ; 3,11 (s) : H en 11 ; 3,78 (s) : $CO_2CH_3$ ; 5,49 (d, J = 10) : H en 1 ; 5,80 (dd) : H en 2 ; 5,8 : H en 6 ; 5,93 (s) : H en 4 ; 7,48 : H méta ; 7,61 (tt) : H para ; 8,08 : H ortho.

| Analyse ($C_{29}H_{32}O_6$ : 476,6) | | |
|---|---|---|
| | C % | H % |
| Calculé | 73,1 | 6,8 |
| Trouvé | 72,9 | 6,9 |

STADE D : 6α-fluoro 9,11β-époxy 16α-méthyl 17α-hydroxy 17β-méthoxycarbonyl androsta 1,4-dièn-3-one

On mélange sous gaz inerte à 20°C, 20 g de produit obtenu au stade C et 100 $cm^3$ d'acétonitrile puis ajoute 2 $cm^3$ d'eau. On refroidit la suspension à -1°/+1°C puis ajoute lentement 17,4 g de N-fluoro N-chlorométhyl triéthylène diamine bis tétrafluoroborate. Après la fin de l'introduction, on maintient sous agitation à -1°/+1°C pendant 1 h puis verse la suspension sur une solution de 400 $cm^3$ d'eau et 10 $cm^3$ d'ammoniaque à 20 %. On introduit sous agitation 0,4 g de métabisulfite de sodium puis poursuit l'agitation pendant 30 mn à température ambiante. On ajoute au besoin une quantité suffisante d'ammoniaque à 20 % pour ajuster le pH à 8 puis essore et lave les cristaux à l'eau puis les sèche. On obtient 16,34 g de produit attendu.
Spectre RMN ($CDCl_3$, 300 MHz, ppm)
0,93 (d) : $CH_3$-CH ; 0,99 (s) : 18-$CH_3$ ; 1,41 (s) : 19-$CH_3$ ; 3,33 (d) : H époxyde ; 3,78 (s) : -$CO_2CH_3$ ; 5,43 (dddd, J HF = 49) : H en 6β ; 6,25 (dd) : H en 2 ; 6,44 (t) : H en 4 ; 6,52 (dd) : H en 1.

| Analyse ($C_{22}H_{27}FO_5$ : 390,45) | | | |
|---|---|---|---|
| | C % | H % | F % |
| Calculé | 67,67 | 6,97 | 4,87 |
| Trouvé | 67,9 | 6,9 | 4,7 |

STADE E : 6α,9α-difluoro 11β,17α-dihydroxy 16α-méthyl 17β-méthoxycarbonyl androsta 1,4-dièn-3-one

On mélange sous gaz inerte 180 $cm^3$ de complexe acide fluorhydrique-diméthylformamide et 18 g de produit obtenu comme décrit au stade D. On agite à 22°C ±3°C pendant 3 heures, puis coule la solution dans un mélange à 0°/+2°C de 1,8 l d'eau et 9 $cm^3$ d'ammoniaque à 22°Bé. En maintenant la température inférieure à 10°C, on ajoute en 30 minutes 290 $cm^3$ d'ammoniaque à 22°Bé, soit la quantité nécessaire pour maintenir le pH à 4,5 ±0,5.

On agite ensuite pendant 1 heure en laissant remonter la température, puis laisse au repos pendant 1 heure. On essore les cristaux, les lave à l'eau à pH neutre et les sèche. On obtient 18,86 g de produit attendu brut que l'on reprend par environ 7 volumes de chloroforme. On porte au reflux et distille environ les 2/3 du chloroforme puis refroidit lentement jusqu'à 0°/+5°C et maintient 1 heure à cette température. On essore et sèche les cristaux. On obtient 18,3 g de produit attendu qu'il est nécessaire de désolvater. On introduit pour cela les cristaux dans 10 volumes d'eau et porte sous agitation à 90°/95°C pendant 30 minutes en distillant le chloroforme. On refroidit, essore les cristaux, les lave à l'eau et les sèche. On obtient 15,8 g de produit. F = 227°C. $\alpha_D^{20}$ = +60° ±1° (c = 1 % DMF). % F Calculé : 9,25, Trouvé : 9 à 9,2.

Le complexe acide fluorhydrique-diméthylformamide utilisé au départ a été préparé comme suit :

On agite pendant 10 mn sous gaz inerte à +19°/+21°C, 210 $cm^3$ de diméthylformamide. On y introduit lentement de l'acide fluorhydrique condensé refroidi à -15°/-20°C, en laissant remonter la température jusqu'à 45°C environ, puis on limite cette remontée de température à +50°/+60°C, par un bain extérieur à -15°/-20°C. On ajoute ainsi au total 250 g d'acide fluorhydrique en 1 h 15 mn. On maintient ensuite la solution pendant quelques minutes sous agitation et gaz inerte, avant l'introduction du stéroïde dans les conditions indiquées plus haut.

**EXAMPLE 2 : 6α,9α-difluoro 11β,17α-dihydroxy 16α-méthyl 17β-carboxy androsta 1,4-dièn-3-one**

On mélange sous gaz inerte, 7,9 g de produit obtenu à l'exemple 1, 75 $cm^3$ de méthanol et 4 $cm^3$ d'eau, puis maintient sous agitation à température ambiante pendant 10 mn. On ajoute en 5 mn, 2,5 g de potasse dans 20 $cm^3$ d'eau

puis porte au reflux lentement. Après 3 h 30 mn au reflux, on refroidit à +50°C et ajoute de l'acide acétique jusqu'à pH - 6, soit environ 3 $cm^3$. On concentre la solution à 40 $cm^3$ environ et refroidit à 20°C puis ajoute de l'eau. On essore les cristaux et les lave au mélange méthanol-eau puis à l'eau et les sèche.

On obtient 7,3 g de produit attendu que l'on purifie par empâtage à chaud dans le méthanol et recristallisation dans l'acétone avec traitement au charbon actif. $\alpha_D^{20}$ = +65°5 (c = 1 % DMF).

| Analyse ($C_{21}H_{26}F_2O_5$) | | | |
|---|---|---|---|
| | C % | H % | F % |
| Calculé | 63,79 | 6,63 | 9,61 |
| Trouvé | 63,7 | 6,6 | 9,3 |

Spectre RMN ($CDCl_3$, 300 MHz, ppm)
1,02 (d) : $CH_3$ ; 1,26 (s) : 18-$CH_3$ ; 1,58 (s) : 19-$CH_3$ ; 4,40 (d,m) : H en 11 ; 5,40 (d,m) : H en 6 ; 6,33 (d) : H en 2 ; 7,18 (d) : H en 1 ; 6,43 (s) : H en 4.
Spectre IR (Nujol),
Absorptions à 3559-3541 $cm^{-1}$ : OH ; 1698-1661 $cm^{-1}$ : C=O et C=O conjuguée ; 1615-1603 $cm^{-1}$ : C=C.

## Revendications

1. Procédé de préparation des composés de formule (I) :

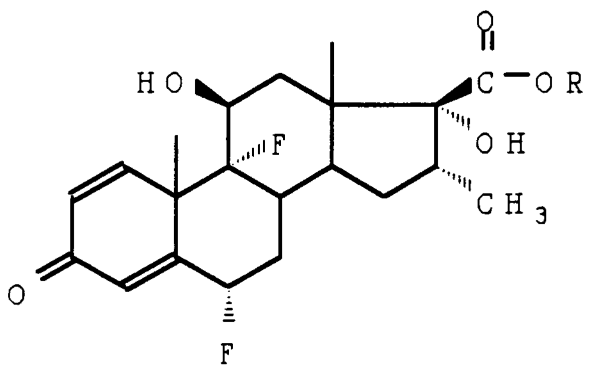

(I)

dans laquelle R représente un atome d'hydrogène ou un reste ester, caractérisé en ce que l'on traite un composé de formule (II) :

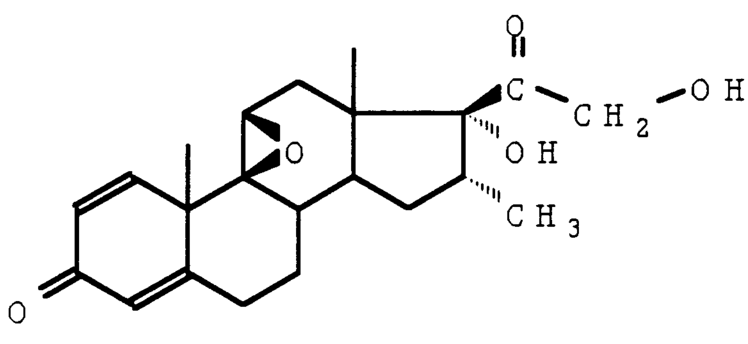

(II)

par un agent de dégradation oxydant, pour obtenir un composé de formule (III) :

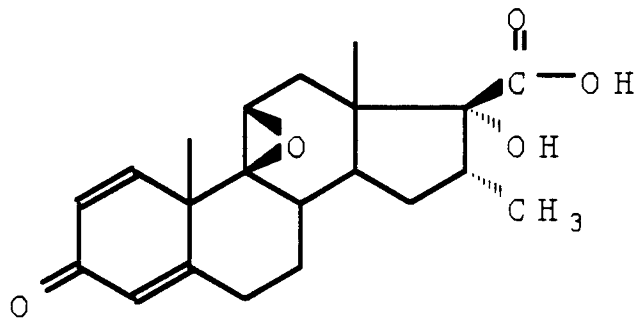

(III)

dont on protège la fonction cétone en 3 sous forme d'éther ou d'ester d'énol et, le cas échéant, la fonction acide en 17β sous forme d'ester, pour obtenir un composé de formule (IV) :

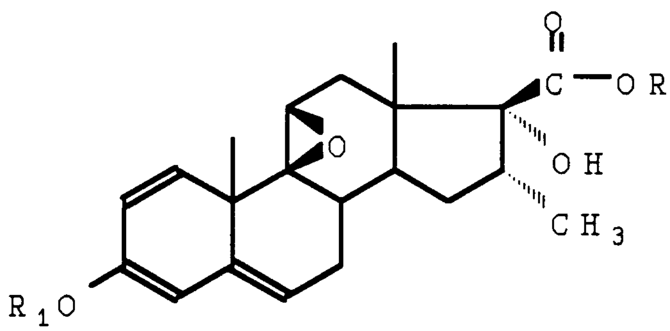

(IV)

dans laquelle R est défini comme précédemment et $R_1$ représente un reste d'éther ou d'ester d'énol, sur lequel on fait agir un agent de fluoration électrophile, pour obtenir un composé de formule (V) :

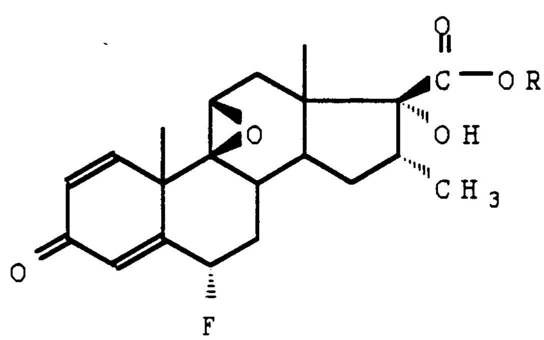

(V)

que l'on traite par un agent de fluoration nucléophile, pour obtenir le composé de formule (I) attendu, que, le cas échéant, lorsque R représente un reste ester, l'on saponifie pour obtenir l'acide correspondant.

2. Procédé selon la revendication 1, caractérisé en ce que l'on protège d'abord la fonction acide du composé de formule (III), pour obtenir un composé de formule (III') :

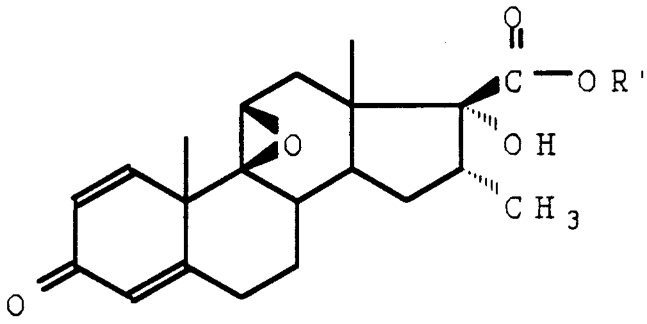

(III')

dans laquelle R' représente un reste ester, dont on protège ensuite la fonction cétone en 3 sous forme d'éther ou d'ester d'énol, pour obtenir un composé de formule (IV) telle que définie à la revendication 1, dans laquelle R a la définition de R' indiquée ci-dessus et poursuit la synthèse comme indiqué à la revendication 1.

3. Procédé selon la revendication 1, caractérisé en ce que l'on protège dans une même opération la fonction cétone en 3 et la fonction acide en 17β respectivement sous forme d'éther d'énol et d'ester, pour obtenir un composé de formule (IV) telle que définie à la revendication 1, dans laquelle R et $R_1$ représentent un même groupement protecteur et poursuit la synthèse comme indiqué à la revendication 1.

4. Procédé selon la revendication 1, caractérisé en ce que l'on protège seulement la fonction cétone en 3 sous forme d'éther ou d'ester d'énol, pour obtenir un composé de formule (IV) telle que définie à la revendication 1, dans laquelle R représente un atome d'hydrogène, puis poursuit la synthèse comme indiqué à la revendication 1.

5. Procédé selon l'une quelconque des revendications 1, 2, ou 4, caractérisé en ce que l'on protège la cétone en position 3 sous forme d'ester d'énol.

6. Procédé selon l'une quelconque des revendications 1, 2 ou 5, caractérisé en ce que l'on protège la fonction acide en position 17β sous forme d'ester d'alkyle.

7. Procédé selon la revendication 1 ou 3, caractérisé en ce que l'on protège les fonctions cétone en position 3 et acide en position 17β respectivement sous forme d'éther et d'ester silylés.

8. Procédé selon l'une quelconque des revendications 1 à 7, caractérisé en ce que l'agent de dégradation oxydant est l'acide periodique.

9. Procédé selon l'une quelconque des revendications 1 à 8, caractérisé en ce que l'agent de fluoration électrophile est le N-fluoro N-chlorométhyl triéthylène diamine bis tétrafluoroborate.

10. Procédé selon l'une quelconque des revendications 1 à 9, caractérisé en ce que l'agent de fluoration nucléophile est le complexe acide fluorhydrique diméthylformamide.

11. Les composés de formule (VI) :

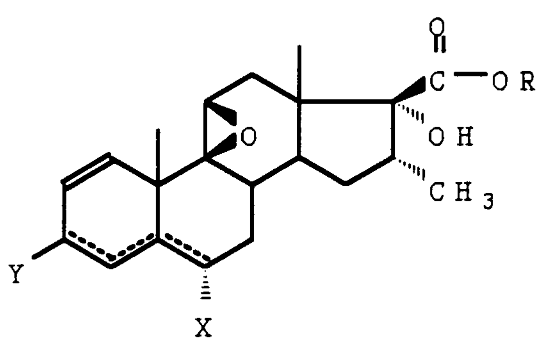

(VI)

dans laquelle R est défini comme à la revendication 1, X représente un atome d'hydrogène ou de fluor et Y associé aux traits pointillés représente un système 3-céto Δ4 ou X représente un atome d'hydrogène et Y associé aux traits pointillés représente un système 3-$OR_1$ Δ3,5, $R_1$ étant défini comme à la revendication 1.

## Claims

1. Preparation process for the compounds of formula (I):

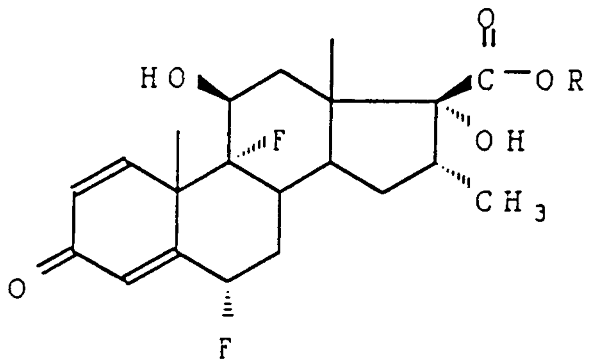

(I)

in which R represents a hydrogen atom or an ester remainder, characterized in that a compound of formula (II):

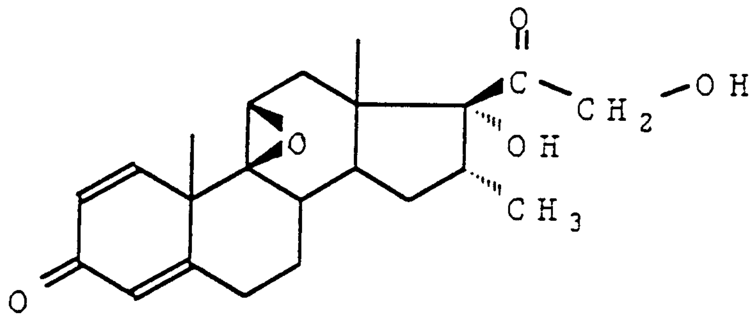

(II)

is treated with an oxidizing degradation agent, in order to obtain a compound of formula (III):

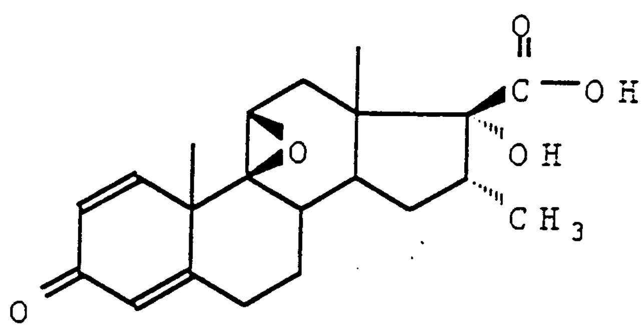

(III)

of which the ketone function in position 3 is protected in the form of the enol ether or ester and, if appropriate, of which the acid function in position 17beta is protected in the form of the ester, in order to obtain a compound of formula (IV):

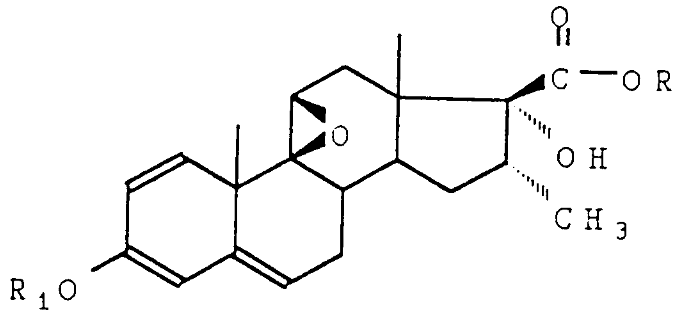

(IV)

in which R is defined as previously and $R_1$ represents a remainder of enol ether or ester, on which an electrophilic fluorination agent is reacted, in order to obtain a compound of formula (V):

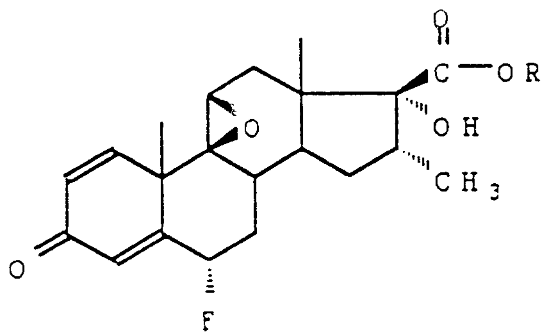

(V)

which is treated with a nucleophilic fluorination agent, in order to obtain the expected compound of formula (I), which, if appropriate, when R represents an ester remainder, is saponified in order to obtain the corresponding acid.

**2.** Process according to claim 1, characterized in that firstly the acid function of the compound of formula (III) is protected, in order to obtain a compound of formula (III'):

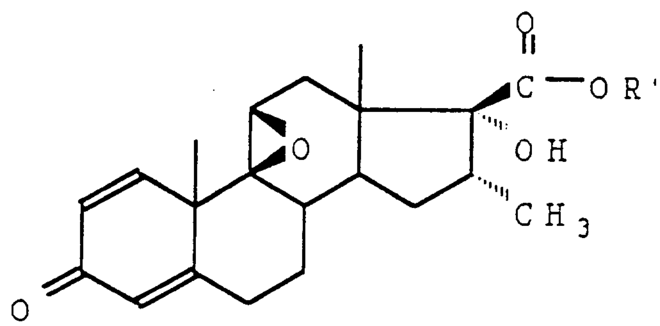

(III')

in which R' represents an ester remainder, of which the ketone function in position 3 is protected in the form of the enol ether or ester, in order to obtain a compound of formula (IV) as defined in claim 1, in which R has the definition of R' indicated above and the synthesis is continued as indicated in claim 1.

**3.** Process according to claim 1, characterized in that in the same operation the ketone function in position 3 and the acid function in position 17beta are protected in the form of the enol ether and ester respectively, in order to obtain a compound of formula (IV) as defined in claim 1, in which R and $R_1$ represent the same protector group and the synthesis is continued as indicated in claim 1.

4. Process according to claim 1, characterized in that only the ketone function in position 3 is protected in the form of the enol ether or ester, in order to obtain a compound of formula (I) as defined in claim 1, in which R represents a hydrogen atom, then the synthesis is continued as indicated in claim 1.

5. Process according to any one of claims 1, 2, or 4, characterized in that the ketone in position 3 is protected in the form of the enol ester.

6. Process according to any one of claims 1, 2 or 5, characterized in that the acid function in position 17beta is protected in the form of the alkyl ester.

7. Process according to claim 1 or 3, characterized in that the ketone function in position 3 and the acid function in position 17beta are protected in the form of the silylated ether and ester respectively.

8. Process according to any one of claims 1 to 7, characterized in that the oxidizing degradation agent is periodic acid.

9. Process according to any one of claims 1 to 8, characterized in that the electrophilic fluorination agent is N-fluoro N-chloromethyl triethylene diamine bis tetrafluoroborate.

10. Process according to any one of claims 1 to 9, characterized in that the nucleophilic fluorination agent is the hydrofluoric acid - dimethylformamide complex.

11. The compounds of formula (VI):

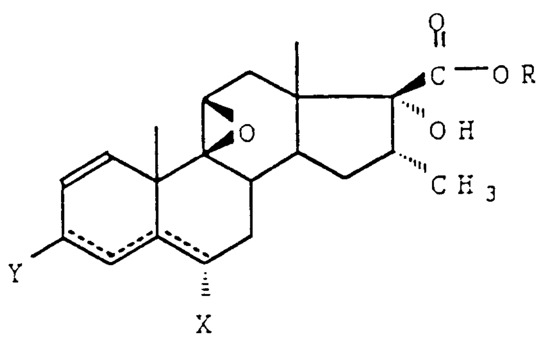

(VI)

in which R is defined as in claim 1, X represents a hydrogen or fluorine atom and Y combined with the dotted lines represents a 3-keto 4 system or X represents a hydrogen atom and Y combined with the dotted lines represents a 3-$OR_1$ 3,5 system, $R_1$ being defined as in claim 1.

**Patentansprüche**

1. Verfahren zur Herstellung von Verbindungen der Formel (I)

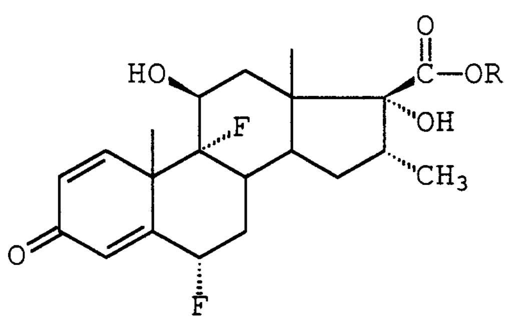

(I)

in der R ein Wasserstoffatom oder einen Esterrest darstellt, dadurch gekennzeichnet, daß man eine Verbindung der Formel (II)

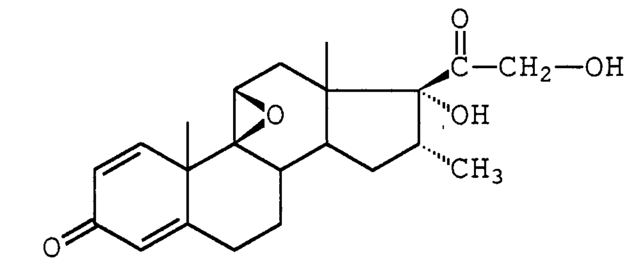

(II)

mit einem Mittel zum oxidativen Abbau behandelt, um die Verbindung der Formel (III)

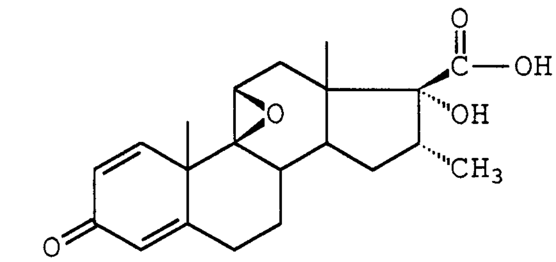

(III)

zu erhalten, bei der man die Ketonfunktion in Position 3 in Form von Enolether oder Enolester und gegebenenfalls die Säurefunktion in 17β in Form von Ester schützt, um eine Verbindung der Formel (IV)

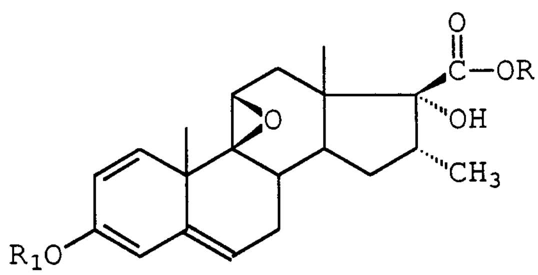

(IV)

zu erhalten, in der R wie oben definiert ist und $R_1$ einen Rest von Enolether oder Enolester darstellt, die man mit einem Mittel zur elektrophilen Fluorierung zur Reaktion bringt, um eine Verbindung der Formel (V)

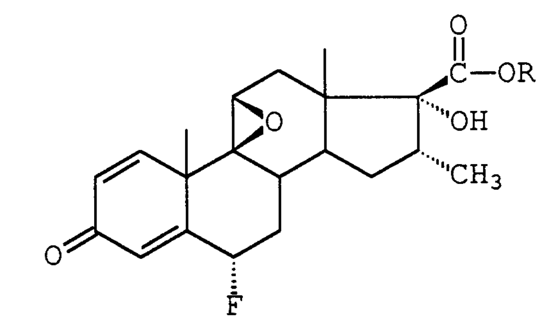

(V)

zu erhalten, die man mit einem Mittel zur nucleophilen Fluorierung behandelt, um die erwartete Verbindung der Formel (I) zu erhalten, die man gegebenenfalls, wenn R einen Esterrest darstellt, verseift, um die entsprechende Säure zu erhalten.

**2.** Verfahren nach Anspruch 1, dadurch gekennzeichnet, daß man zunächst die Säurefunktion der Verbindung der Formel (III) schützt, um eine Verbindung der Formel (III')

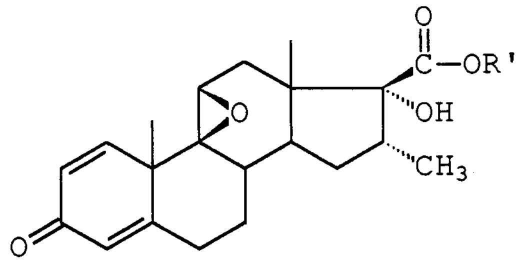

(III')

zu erhalten, in der R' einen Esterrest darstellt, bei der man anschließend die Ketonfunktion in Position 3 in Form von Enolether oder Enolester schützt, um eine wie in Anspruch 1 definierte Verbindung der Formel (IV) zu erhalten, in der R die oben angegebene Bedeutung von R' besitzt, und man anschließend die Synthese wie in Anspruch 1 angegeben fortsetzt.

**3.** Verfahren nach Anspruch 1, dadurch gekennzeichnet, daß man in einer gleichen Operation die Ketonfunktion in Position 3 und die Säurefunktion in Position 17β in Form von Enolether bzw. Ester schützt, um eine wie in Anspruch 1 definierte Verbindung der Formel (IV) zu erhalten, in der R und $R_1$ eine gleiche Schutzgruppe aufweisen, und man anschließend die Synthese wie in Anspruch 1 angegeben fortsetzt.

**4.** Verfahren nach Anspruch 1, dadurch gekennzeichnet, daß man lediglich die Ketonfunktion in Position 3 in Form von Enolether oder Enolester schützt, um eine wie in Anspruch 1 definierte Verbindung der Formel (IV) zu erhalten, in der R ein Wasserstoffatom darstellt, und man anschließend die Synthese wie in Anspruch 1 angegeben fortsetzt.

**5.** Verfahren nach irgendeinem der Ansprüche 1, 2 oder 4, dadurch gekennzeichnet, daß man das Keton in Position 3 in Form von Enolester schützt.

**6.** Verfahren nach irgendeinem der Ansprüche 1, 2 oder 5, dadurch gekennzeichnet, daß man die Säurefunktion in Position 17β in Form von Alkylester schützt.

**7.** Verfahren nach Anspruch 1 oder 3, dadurch gekennzeichnet, daß man die Ketonfunktion in Position 3 und die Säurefunktion in Position 17β in Form von Silylether bzw. Silylester schützt.

**8.** Verfahren nach irgendeinem der Ansprüche 1 bis 7, dadurch gekennzeichnet, daß das Mittel zum oxidativen Abbau Periodsäure ist.

**9.** Verfahren nach irgendeinem der Ansprüche 1 bis 8, dadurch gekennzeichnet, daß das Mittel zu elektrophilen Fluorierung N-Fluor-N-clormethyl-triethylendiamin-bis-tetrafluorborat ist.

**10.** Verfahren nach irgendeinem der Ansprüche 1 bis 9, dadurch gekennzeichnet, daß das Mittel zu nucleophilen Fluorierung der Fluorwasserstoffsäure-Dimethylformamid-Komplex ist.

**11.** Verbindungen der Formel (VI)

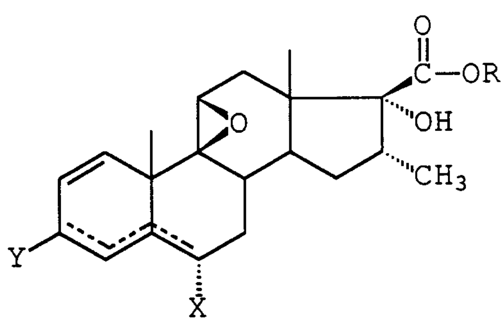

(VI)

in der R wie in Anspruch 1 definiert ist, X ein Wasserstoffatom oder ein Fluoratom darstellt und Y, verbunden mit

den punktierten Linien, ein System 3-Keto Δ4 bedeutet, oder X ein Wasserstoffatom darstellt und Y, verbunden mit den punktierten Linien, ein System 3-$OR_1$ Δ3,5 bedeutet, worin $R_1$ wie in Anspruch 1 definiert ist.